# FASCICULE DE BREVET EUROPEEN

(11) **EP 2 121 131 B1**
(45) Date de publication et mention de la délivrance du brevet: **22.08.2012**
(21) Numéro de dépôt: 08787822.9
(22) Date de dépôt: 20.03.2008
(51) Int. Cl.: A61N 5/06

(54) **DISPOSITIF D'IRRADIATION PAR RAYONNEMENT D'ENERGIE PHOTONIQUE MODULEE.**
BESTRAHLUNGSGERÄT MIT MODULIERTER PHOTONENENERGIEBESTRAHLUNG
IRRADIATION DEVICE USING A MODULATED PHOTONIC-ENERGY RADIATION

(30) Priorité: 21.03.2007 FR 0702068
(43) Date de publication de la demande: 25.11.2009
(73) Titulaire: FOREFRONT INFRARED & Technologies sprl, 1340 Ottignies Louvain La Neuve (BE)
(72) Inventeur: BREDA, Jean-Pierre, F-73490 La Ravoire (FR); CORDEMANS de MEULENAER, Eric, BE-1970 Wezembeek (BE)
(74) Mandataire: Vanhalst, Koen
(86) Numéro de dépôt international: PCT/FR2008/000372
(87) Numéro de publication internationale: WO 2008/135658

(56) Documents cités:
- WO-A-92/20403
- WO-A-95/19810
- DE-A1- 3 134 953
- US-A- 5 800 481

## Description

### Domaine technique de l'invention

L'invention concerne un dispositif d'irradiation par rayonnement d'énergie photonique, comprenant un module émetteur photonique de fréquence nanométrique, de longueur d'onde prédéterminée.

Ce type de dispositif d'irradiation utilise un module émetteur à diodes LED ou laser pour des applications de photo bio stimulation (PBS), notamment pour stimuler ou modifier une réaction de défense des cellules de l'organisme, en apportant notamment un soulagement important des douleurs d'origines inflammatoires et physiologiques.

Les cellules vivantes réagissent de manières différentes aux sollicitations électromagnétiques, chimiques, thermiques ou mécaniques.
L'analyse protéomique a permis d'identifier des séquences de protéines impliquées dans les mécanismes de stress et de défense, ainsi que dans des réactions de réponses à la lumière. Malgré la complexité due à l'ensemble des participants impliqués dans la séquence mise en jeu, de tels mécanismes de réaction sont rapides (de l'ordre de quelques minutes).
Il est maintenant acquis que des combinaisons spécifiques de chemins réactionnels, souvent inter agissants entre eux, mènent à des modes de transcription distincts au cours des phénomènes de stress (on définit par stress, des conditions d'application d'énergies au milieu vivant qui ne sont pas celles que les cellules rencontrent de manière physiologique habituelle). L'application d'un stress énergétique doux permet tout à la fois de modifier les mécanismes physiologiques cellulaires, mais également, de par l'application envisagée, d'en influencer les cinétiques et les orienter vers le but recherché.

Ce réseau régulateur permet, dans le cas des plantes par exemple, de fournir des réponses très finement adaptées aux différents stimuli, et qui gèrent le développement des cellules, tissus et organes.
La majorité des séquences protéiniques générées par les conditions de stress impliquent en même temps des réactions de défense. Des séquences similaires sont impliquées dans les mêmes mécanismes stress - défense dans les cellules animales et les levures.
Les mécanismes physiologiques (synthèse de protéines, stimulation et modification des mécanismes de synthèses endogènes de métabolites, modification des facteurs sigma, implication de séquences génétiques différentes, poration, etc....) modifiés par l'application de ce stress peuvent être, selon les énergies mises en jeu, temporaires et/ou réversibles, ou mener à la destruction sélective de certaines populations, alors que d'autres populations sont modifiées différemment, menant à leur croissance ou à leur décroissance.
Les réponses cellulaires dépendent en plus d'autres facteurs tels que la fréquence, la puissance et la durée de l'irradiation, de la nature des milieux de couplage entre le module émetteur et le milieu traité.
Par la combinaison originale de trois domaines d'ondes électromagnétiques, la présente technologie permet d'orienter les effets selon les plages de fréquences utilisées et les paramètres dont elles dépendent.

### État de la technique

Le document US 6602275 décrit un dispositif de photothérapie faisant usage de trois séries de diodes LED de différentes longueurs d'ondes (470nm, 630nm, et 880nm). Un circuit de commande est branché entre l'alimentation et les diodes LED, et comporte un processeur électronique connecté par l'intermédiaire d'interrupteurs et d'un convertisseur numérique analogique à des amplificateurs opérationnels destinés à piloter des transistors FET associés aux différentes séries de diodes LED. L'ensemble permet d'ajuster le niveau de puissance, la durée et la vitesse de répétition et de séquence des impulsions d'alimentation.

Les documents US No. 3.280.816 , FR 1.342.772 et FR 1.501.984 concernent l'émission d'électrons dans un large spectre de fréquences 15MHz à 300MHz, nécessitant une énorme énergie (afin de réaliser un plasma) d'hyper fréquences et de fréquences HF. A ces fréquences et ces puissances, l'effet thermique est important.

Le document US No. 5,584,863 se rapporte à un appareillage de traitements électromagnétiques qui utilise des hautes fréquences, à hautes énergies, mais sans effet thermique.

Le brevet US 5,800,481 décrit l'utilisation de deux oscillateurs basse fréquence reliés en entrée d'un circuit logique ET, le premier oscillateur fonctionnant à 2,4Hz et le second fonctionnant à 0,5Hz. L'utilisation de ces fréquences permet de stimuler les processus corticaux et d'agir sur le système nerveux.

La photo bio stimulation (PBS) dans le domaine rouge à infrarouge proche (630- 1000 nm) permet d'accélérer la guérison des blessures, d'améliorer la récupération de blessures ischémiques cardiaques et d'atténuer la dégénérescence d'un nerf optique blessé (voir brevet US No 2004215293). La photothérapie LED a été également utilisée pour accélérer l'éclosion et diminuer le taux de mortalité des oeufs de poule.

### Objet de l'invention

L'objet de l'invention concerne un dispositif d'irradiation permettant une meilleure pénétration du rayonnement électromagnétique dans le milieu à traiter, en stimulant de manière optimale les réactions physiologiques des cellules de l'organisme.

Le dispositif d'irradiation par rayonnement d'énergie photonique comporte un module émetteur photonique de fréquence nanométrique et est caractérisé en ce que le module émetteur est relié à des moyens de modulation assurant un premier découpage à basse fréquence BF, et un deuxième découpage à radiofréquence RF pour obtenir une suite de créneaux à basse fréquence BF découpés à la radiofréquence RF.

Le premier découpage à basse fréquence BF a pour but la stimulation des cellules, le deuxième découpage à radiofréquence RF a pour but la dépolarisation des cellules.

Selon un développement de l'invention, le découpage à basse fréquence BF a une plage de fréquence comprise entre 5 Hz et 50 Hz, et de préférence entre 10 Hz et 20 Hz. Le découpage à radiofréquence RF a une plage de fréquence comprise entre 500 kHz et 10.000 kHz, et de préférence entre 1.000 kHz et 1.500 kHz.

Selon un développement, le module émetteur photonique, de longueur d'ondes prédéterminée, est connecté à une source de courant continu par un transistor pour former un circuit de découpage.

Le circuit de découpage peut comporter un circuit logique ET aux entrées duquel sont connectés un premier oscillateur à basse fréquence et un deuxième oscillateur à radiofréquence, et que la sortie du circuit logique ET est branchée à la grille du transistor pour moduler l'alimentation à double découpage par les deux fréquences desdits premier et deuxième oscillateurs.

Selon un développement, le module émetteur est doté d'une série de diodes électroluminescentes ou laser à rayonnement proche de l'infrarouge, et du type GaAlAs.

Le procédé d'irradiation, utilisant le dispositif, met en jeu la combinaison originale de trois domaines d'ondes électromagnétiques, à savoir :
- une émission de photons, de fréquence nanométrique,
- un découpage de l'émission nanométrique par basse fréquence pour stimuler les cellules et permettre une action plus efficace des ondes nanométriques,
- un découpage de l'émission nanométrique par radiofréquence afin de dépolariser les cellules, permettant ainsi une pénétration plus efficace des photons.

Le rayonnement des photons de fréquence nanométrique et modulé par la BF et la RF est émis par des diodes LEDs ou des diodes laser. Le signal résultant est composé d'une émission de photons par onde électromagnétique de plusieurs centaines de THz (Tera-Hertz = 10¹² Hz) découpée en impulsions par une basse fréquence et une radiofréquence et modulée par les harmoniques de ces deux fréquences.

Ce rayonnement de photons s'effectue dans des conditions énergétiques douces.

C'est la combinaison du découpage et de la modulation de la fréquence nanométrique par deux fréquences et la modulation par leurs harmoniques qui provoque l'effet recherché. La propagation de ces ondes se fait sans contact direct avec la cible que représentent les organismes vivants, dans des conditions énergétiques douces propres aux mécanismes fondamentaux du vivant et sans apport ni utilisation d'aucun agent ou réactifs de nature chimique ou autre. L'avantage de cette technique réside dans le fait qu'elle permet une pénétration plus aisée de l'onde, tout en n'utilisant qu'une faible énergie.

Les effets observés par l'application du procédé selon l'invention sont, entre autres :
- la modification de la germination des graines, de la levée de semis et la croissance des plantes,
- les effets physiologiques de soulagement des douleurs musculaires, foulures, maux de dos, douleurs post-traumatiques.

### 1) Effets physiques de pénétration de l'onde: L'effet physique est dû à la modulation RF.

Le choix s'est porté sur un oeuf, milieu représentatif d'un milieu vivant, et sur une irradiation infrarouge proche de 875 nanomètres.

L'émetteur IR délivre 100 mW/cm², il est placé à 20 cm de l'oeuf. La mesure se fait au centre de l'oeuf, à l'aide d'un capteur IR.

| traitement | Mesure (mW/cm²) | Facteur d'atténuation | Effet de pénétration de l'onde IR |
|---|---|---|---|
| IR seuls | 0,5 | 200 | Pénétration faible |
| IR avec modulation BF | 0,5 | 200 | Pénétration faible |
| IR avec modulation RF | 10 | 10 | RF est la porteuse de l'IR |
| IR avec modulation( BF+RF) | 10 | 10 | RF permet la pénétration, la BF l'effet biocellulaire |

### 2) Effets de réponse bio cellulaires : l'effet est dû aux basses fréquences BF

### a) traitement des graines de radis:

**essais de germination après exposition des graines durant 15 minutes,**

| traitement | Germinaison | Croissance |
|---|---|---|
| Témoin | 3 jours | |
| IR seuls | 3 jours | Identique au témoin |
| IR modulation(BF+RF) | 5 jours | Retard (qui se rattrape par rapport aux autres après 3-4 jours (J0 + 8-9) : donc croissance plus rapide en finale. Le plant est plus vigoureux, les feuilles sont déjà découpées et plus vertes après 8 jours (J0 + 14) |

ensuite, après plantation, irradiation de 15 min/jour

### b) traitement des graines de concombre

Non irradiation avant plantation, mais après plantation, irradiation des pots tous les jours15'/jour

| traitement | Germinaison | Croissance |
|---|---|---|
| Témoin | 8 jours | |
| IR seuls | 8 jours | Croissance plus rapide que témoin |
| IR modulation(BF+RF) | 6 jours | Croissance plus rapide, mais également croissance plus vigoureuse (feuilles plus vertes) |

### c) traitement des graines de salade et de persil

Les graines de persil sont difficiles à faire germer (il faut généralement 40 jours pour les faire germer)
Résultats après 21 jours :

**Germination**

| traitement | Germinaison persil | Germinaison salade |
|---|---|---|
| IR seuls | Pas de germinaison | Faible germinaison |
| témoin | Pas de germinaison | Germinaison moyenne |
| IR modulation(BF+RF) | Germinaison très nombreuse | Germinaison très nombreuse |

15 ' irradiation avant plantation, mais après plantation, irradiation des pots tous les jours durant15'/jour

Les effets sur les graines de persil et de salades sont différents :
L'irradiation pulsée fait germer plus les graines
L'irradiation continue fait germer moins de graines de salade, mais celles qui sont germées, lèvent plus haut que celles irradiées en pulsé.
Par contre, pour le persil, qui est connu pour mal pousser, après un long délai, l'irradiation pulsée seule permet la levée ; ni l'irradiation continue, ni le semis naturel ne permettent la germination rapide.

**Croissance**

| traitement | Croissance persil | Croissance salade |
|---|---|---|
| IR seuls | - | Montée plus vigoureuse |
| Témoin | - | Montée moyenne |
| IR modulation(BF+RF) | -montée uniquement sous conditions pulsées | Montée moyenne |

### 3) Effets sur la perméabilisation des cellules par test Bleu Alamar

La perméabilisation de la membrane cellulaire a été analysée par transfert d'une molécule à l'intérieur des cellules.

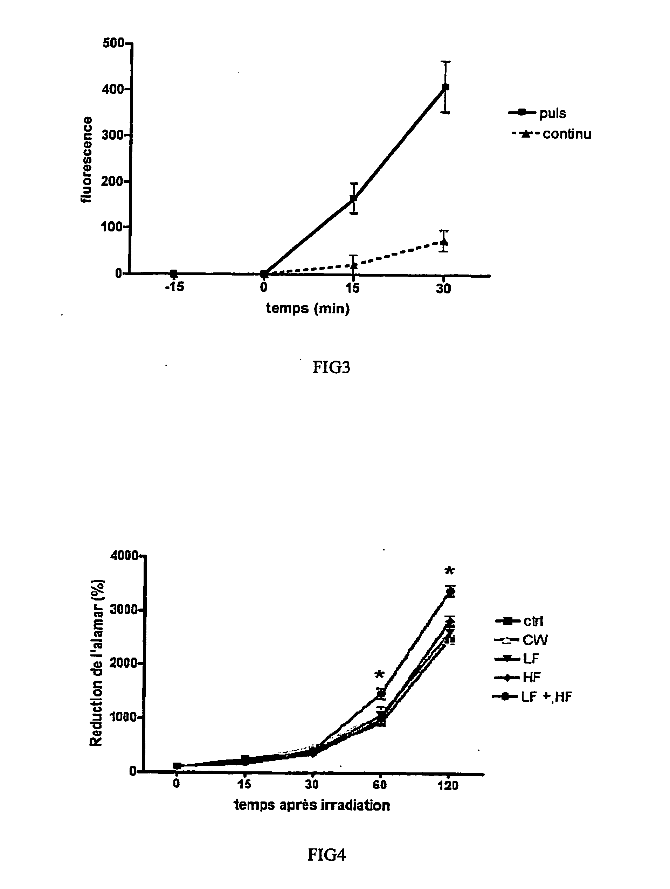

La figure 3 illustre un test au Bleu Alamar. L'Alamar est un composé qui change de couleur lorsqu'il est réduit à l'intérieur de la cellule, il passe alors de la couleur bleu à la couleur rouge en devenant fluorescent rendant possible une lecture de la fluorescence émise par un fluorimètre. Le graphique de la figure 3 correspond à un test au Bleu Alamar sur des thyrocytes humains en comparant le mode doublement pulsé des I.R. (modulés à 12 Hz et 1.200 kHz) avec le mode continu des I.R. L'analyse de la luminescence est réalisée durant 15 minutes d'irradiation, puis après arrêt de cette dernière.

Le mode pulsé démontre que le trafic transmembranaire est très important aussitôt l'irradiation stoppée.

La figure 4 illustre les effets sur la réduction du Bleu Alamar dans des thyrocytes de porc, seul le groupe LF+HF, correspondant au mode pulsé des I.R. modulés à basse fréquence 12 Hz et à radiofréquence 1.200 kHz est significativement différent.

### 4) Mesure de la modification transépithéliale « TRE » (TER ou TEER en anglais)

Cette mesure permet d'obtenir le taux de perméabilité entre cellules adjacentes, illustrant la possibilité de passage des molécules d'une cellule à l'autre.

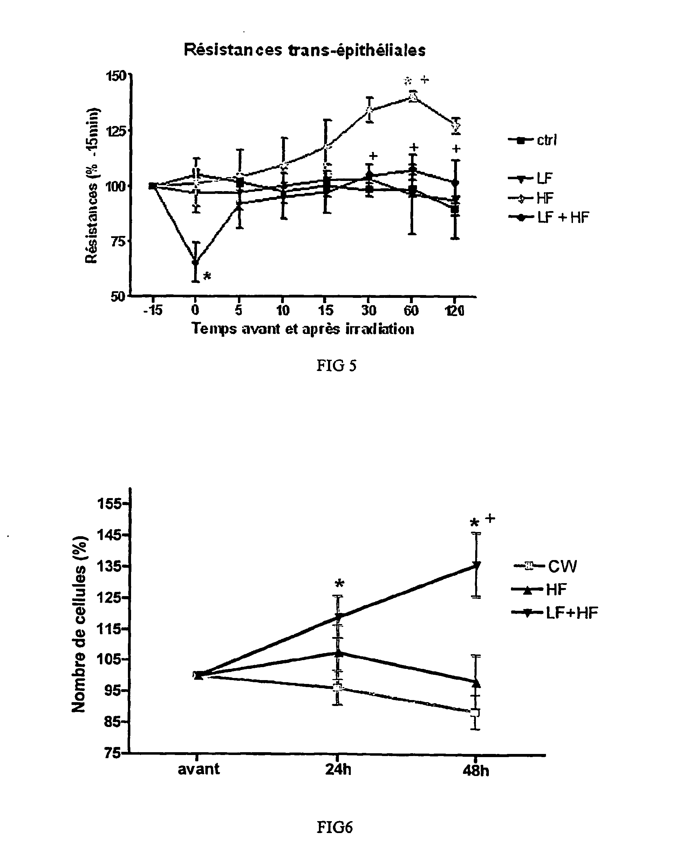

La figure 5 illustre les effets sur les TRE de thyrocytes de porc cultivés en système bicaméral. Ctrl correspond à la mesure des cellules non irradiées, LF correspond à une irradiation I.R. modulée à basse fréquence (12 Hz), HF correspond à une irradiation I.R. modulée à radiofréquence (1.200 kHz), LF+RF correspond à une irradiation I.R. doublement modulée (12 Hz et 1.200 kHz). Les mesures sont effectuées durant 15 minutes d'irradiation, puis après arrêt de cette dernière.

Les résultats montrent que seul le mode pulsé, au cours de l'irradiation elle-même, désactive les fonctions de barrière de la membrane cellulaire (la résistance transépithéliale correspond à une autre vue de la perméabilité de la membrane, ainsi plus la perméabilité est grande, plus la TRE est faible) au cours de l'irradiation. Dès que l'irradiation cesse, les fonctions de barrière entre les cellules sont réactivées, redonnant à chaque cellule son autonomie et son dynamisme interne. Le retour à des conditions physiologiques normales se fait de façon extrêmement rapide dans le cas de la double modulation. Les résultats indiquent une action au niveau intercellulaire, sans besoin d'appliquer des irradiations de longue durée.

### 5) Effets sur la prolifération de cellules épithéliales de thyroïde de rats (lignée cellulaire PCCL3)

Les cellules ayant subi un stress cellulaire dû à la congélation et la décongélation, mettent , en conditions de cultures optimales telles que celles indiquées par Fusco A, Berlingieri MT, Di Fiore PP, Portella G, Grieco M, Vecchio G (1987 One- and two-step transformations of rat thyroid epithelial cells by retroviral oncogenes. Mol Cell Biol 7:3365-3370.) de une à deux semaines avant de reprendre et ensuite se multiplier de façon normale.

La figure 6 illustre le pourcentage de cellules à 24 et 48 heures après décongélation de la lignée PCCL3. Les cellules sont irradiées 15 minutes puis mises en culture. CW correspond à une irradiation I.R. non modulée, HF correspond à une irradiation I.R. modulée à radiofréquence (1.200 kHz) et LF+HF correspond à une irradiation I.R. doublement modulée (12 Hz et 1.200 kHz). Seul le groupe LF+HF est significatif. La diminution du nombre de cellules dans le groupe CW signifie que ce type d'irradiation ne permet pas aux cellules de retrouver une vigueur normale beaucoup d'entre elles étant trop fragilisées ne survivent pas. Seul le mode de double modulation synergise une prolifération significative, dès l'irradiation. Ceci permettrait d'envisager de pouvoir utiliser la présente technologie pour initier rapidement la prolifération de lignées traditionnellement fragiles et difficiles à cultiver.

Ces résultats indiquent que la double modulation induit des effets différents de ceux obtenus par la simple modulation à basses fréquences ou à radiofréquences. Nous assistons à l'initiation de mécanismes cellulaires fondamentaux.

### Description sommaire des dessins

D'autres avantages et caractéristiques ressortiront plus clairement de la description qui va suivre d'un mode de réalisation de l'invention donné à titre d'exemple non limitatif et représenté au dessin annexé, dans lequel :
- la figure 1 représente le schéma simplifié du circuit électronique du dispositif d'irradiation selon l'invention ;
- la figure 2 montre la forme du train d'impulsions en sortie du circuit de découpage.
- Les figures 3 à 6 représentent des résultats liés à l'utilisation du dispositif selon l'invention.

### Description détaillée de l'invention

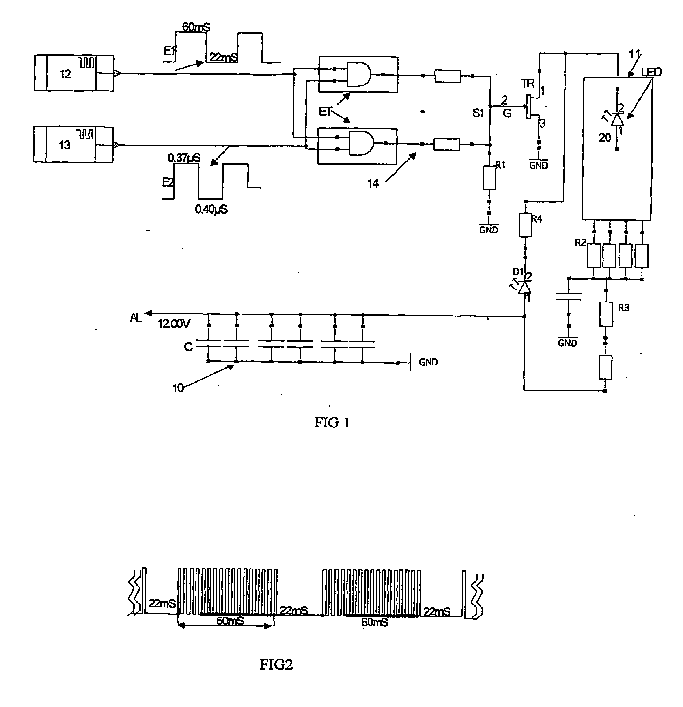

En référence à la figure 1, un dispositif d'irradiation 10 par rayonnement d'énergie photonique modulée, est composée d'un module émetteur 11 photonique capable de délivrer une onde électromagnétique nanométrique, notamment de plusieurs centaines de THz (Tera-Herz 10¹²).

Le module émetteur 11 est relié à des moyens de modulation assurant un premier découpage à basse fréquence BF dont la plage de fréquence est, de préférence, comprise entre 5 Hz et 50 Hz ou plus particulièrement entre 10 Hz et 20 Hz, et un deuxième découpage à radiofréquence RF dont la plage de fréquence est, de préférence, comprise entre 500 kHz et 10.000 kHz ou plus particulièrement entre 1.000 kHz et 1.500 kHz. Cette double modulation permet d'obtenir une suite de créneaux à basse fréquence BF découpés à la radiofréquence RF.

Le module émetteur photonique 11 est formé par une pluralité de diodes électroluminescentes LED ou laser (une seule est représentée sur la figure 1) à rayonnement proche de l'infrarouge, par exemple du type GaAlAs.

Selon un mode de réalisation particulier, les diodes LED sont alimentées par une source de courant continu AL à très basse tension (par exemple entre 3V et 12V) à travers un transistor TR de commande. De préférence, le module émetteur émet de l'énergie lumineuse dans le domaine des ultraviolets, visibles ou infrarouge (proche ou lointain).

Le transistor TR de commande peut être un transistor à effet de champ dont la grille est connectée électriquement à la sortie S1 d'au moins un circuit logique ET, l'ensemble formant un circuit de découpage 14. Le transistor TR et le circuit logique ET peuvent bien entendu être remplacés par tout autre composant pour assurer le découpage des impulsions d'alimentation.

Un premier oscillateur 12 à basse fréquence, et un deuxième oscillateur 13 à radiofréquence sont reliés à l'entrée du circuit logique ET. La sortie du circuit logique ET est branchée à la grille du transistor TR afin de moduler l'alimentation à double découpage par les deux fréquences BF et RF des premier et deuxième oscillateurs.

Ainsi lorsque les signaux des premier et deuxième oscillateurs parviennent aux entrées respectives du circuit logique ET, la sortie du circuit logique ET délivre une suite de créneaux sous forme d'impulsions à basse fréquence BF découpés à la radiofréquence RF.

À titre d'exemple, le premier oscillateur 12 de fréquence 12 Hz délivre des impulsions E1 carrées de 60ms toutes les 82ms (Duty Cycle = 73%). Le deuxième oscillateur 13 de fréquence 1.230 kHz délivre des impulsions E2 de 0,37 microsecondes toutes les 0,77 microsecondes (Duty Cycle = 48%).

La figure 2 illustre le train d'impulsions en sortie S1 du circuit de découpage 14, soit un total de 77922 impulsions de 0,37microsecondes toutes les 0,77 microsecondes pendant 60ms et renouvelé toutes les 82ms, ainsi les impulsions sont générées pendant 60ms puis la sortie du circuit S1 ne génère plus rien pendant 22ms avant de réaliser un nouveau cycle d'impulsions, etc.

Ces impulsions de découpage sont appliquées à la grille G du transistor TR, laquelle est connectée à la masse par une résistance R1. L'une des électrodes du transistor TR est en liaison avec la masse, et l'autre électrode est connectée à la cathode des diodes LED émettrices. L'anode des diodes LED est branchée à la source de courant continu AL, soit directement, soit à travers des résistances R2, R3. La source de courant continu AL peut être constituée par une batterie ou un convertisseur AC-DC associé à un circuit de régulation à basse tension.

Les diodes LED émettent des rayonnements de mêmes longueurs d'ondes, et sont avantageusement connectées en série dans une même branché. Plusieurs branches peuvent être reliées en parallèle en fonction du nombre de diodes LED.

Une diode de signalisation D1 marche-arrêt est connectée en série avec une résistance R4 entre la source de courant continu AL et la cathode des diodes LED du module émetteur 11. Des condensateurs C sont branchés en parallèle entre la source AL et la masse.

La présence du circuit de découpage 14 piloté par les deux oscillateurs 12, 13 assure l'alimentation pulsée des diodes LED pour l'émission de l'onde électromagnétique nanométrique de plusieurs centaines de THz, laquelle est découpée en impulsions par la basse fréquence de 12 Hz et la radiofréquence de 1.230kHz. Ce double découpage génère des harmoniques qui modulent l'émission principale en THz aux fréquences de 12 Hz et 1.230 kHz.

La présente invention n'est pas limitée à l'utilisation d'un circuit de découpage et d'une porte logique ET. En effet, la double modulation par découpage à basse fréquence BF et à radiofréquence RF peut être obtenue sans passer par l'utilisation d'une porte logique ET. À titre d'exemple le dispositif d'irradiation peut comporter un oscillateur à radiofréquence RF bloqué et débloqué par un oscillateur à basse fréquence BF agissant sur l'oscillateur à radiofréquence RF.

Selon un autre exemple, le dispositif peut comporter un oscillateur à radiofréquence piloté par un microcontrôleur appliquant lui-même le découpage à basse fréquence en comptant les impulsions de l'oscillateur à radiofréquence. Ainsi, en reprenant l'exemple de la figure 2, le microcontrôleur permet d'obtenir un train d'impulsions comportant 77922 impulsions de 0,37microsecondes toutes les 0,77 microsecondes pendant 60ms et renouvelé toutes les 82ms. Les impulsions sont générées pendant 60ms puis le microcontrôleur stoppe l'oscillateur pendant 22ms avant de réaliser un nouveau cycle d'impulsions, etc.

On a constaté que l'utilisation de telles ondes électromagnétiques nanométriques découpées et modulées par une basse fréquence et une radiofréquence, permet également de stimuler les cellules de l'organisme, en apportant notamment un soulagement important des douleurs d'origines inflammatoires et physiologiques (foulures, maux de dos, douleurs post-traumatiques, etc....). Le découpage à basse fréquence (12 Hz) favorise la stimulation des cellules et permet une action plus efficace des ondes électromagnetiques dans les cellules, et le découpage à radiofréquence (1.230 kHz) permet une dépolarisation du milieu à traverser. L'émission du rayonnement produite par les diodes LED a une fréquence centrale de l'ordre de 875nm (343THz) qui se situe à la limite du visible et de l'infrarouge. La puissance moyenne émise est faible, environ de 0,3W/cm²_{.}

En fonction de l'effet recherché pour une application prédéterminée, il est possible de modifier les valeurs des fréquences de découpage. La fréquence BF du premier oscillateur peut être comprise de préférence entre 5 Hz et 50 Hz ou plus particulièrement entre 10 et 20 Hz, tandis que la fréquence RF du deuxième oscillateur est comprise entre 500 kHz et 10.000 kHz ou plus particulièrement entre 1.000 kHz et 1.500 kHz.

Les réponses cellulaires dépendent en plus d'autres facteurs, tels que la fréquence, la puissance et la durée de l'irradiation, de la nature des milieux de couplage entre le module émetteur 11 et le milieu traité. Les séquences d'irradiation et leur répétition au cours du temps jouent également un rôle dans la modulation de la stimulation des réponses cellulaires.

Les diodes LED du module émetteur 11 qui subissent la double modulation peuvent être remplacées par d'autres composants électroluminescents, notamment des diodes laser, pour obtenir des effets différents, notamment sur la germination des graines ou semis.

Les domaines d'applications de la présente technologie de photo-biostimulation (à savoir l'utilisation de fréquences nanomètriques découpées et modulées par une fréquence BF et RF) sont les suivants:
- stimulation des réponses cellulaires des milieux biologiques (cellules prokaryotes, eukaryotes, bactéries, levures, moisissures... )
- traitement des cellules dans les domaines identiques à ceux de la photothérapie et de la photophérèse ,
- amélioration des techniques utilisées en biotechnologies cellulaires (poration, transfection),
- croissances cellulaires (cultures)
- optimalisation du fonctionnement des milieux nutritifs.
- croissance des embryons, éclosion des oeufs,
- régénération des fractures osseuses, de la peau, de cartilages, tendons, muscles, recollement des structures nerveuses sectionnées
- guérison des blessures
- traitement de l'arthrite et autres inflammations
- application en vétérinaire
- semis et croissance des plantes
- action dans les milieux gazeux (air, oxygène... ou autres mélanges de gaz), liquide, ou sur toutes masses vivantes
- bio-réacteurs
- application dans le domaine du traitement des eaux, des boues et de l'épuration.

Un tel dispositif permet de traiter de manière sélective ou spécifique certaines cellules et organismes (cellules de moisissures, levures, champignons, algues, bactéries, plantes, cellules animales ou humaines). Les cellules peuvent se trouver dans un milieu liquide en batch ou dans un fluide en circulation. La perméabilisation de la membrane cellulaire permet de faire pénétrer de manière douce une protéine ou une molécule chimique à l'intérieur de la cellule. Lorsque les pores des cellules sont ouverts, des transferts de molécules exogènes vers l'intérieur des cellules et des métabolites endogènes vers l'extérieur peuvent se dérouler, les cellules pouvant perdre ainsi une grande partie de leur ATP tout en restant vivantes.

Selon les paramètres utilisés, le dispositif permet de détruire les cellules et organismes ou de favoriser les développements cellulaires.

La présente technologie ouvre le champ à des applications thérapeutiques nouvelles, telles la transfection de gènes au niveau du noyau ou de l'induction de mécanismes cellulaires secondaires mettant en jeu l'artillerie des signaux cellulaires. Un tel dispositif permet aussi de faciliter l'assimilation dans le cas où les fonctions sont déficientes.

## Revendications

1. Dispositif d'irradiation par rayonnement d'énergie photonique comportant un module émetteur (11) photonique de longueur d'onde nanométrique, **caractérisé en ce que** le module émetteur (11) est relié à des moyens de modulation assurant un premier découpage à basse fréquence (BF), et un deuxième découpage à radiofréquence (RF), pour obtenir une suite de créneaux à basse fréquence (BF) découpés à la radiofréquence (RF).

2. Dispositif d'irradiation selon la revendication 1, **caractérisé en ce que** la plage de fréquence du premier découpage à basse fréquence (BF) est comprise entre 5 Hz et 50 Hz, et que la plage de fréquence du deuxième découpage à radiofréquence (RF) est comprise entre 500 kHz et 10.000 kHz.

3. Dispositif d'irradiation selon la revendication 2, **caractérisé en ce que** la plage du deuxième découpage à radiofréquence (RF) est comprise entre 1.000 kHz et 1.500 kHz.

4. Dispositif d'irradiation selon l'une des revendications 2 et 3, **caractérisé en ce que** la plage du premier découpage à basse fréquence (BF) est comprise entre 10Hz et 20Hz.

5. Dispositif d'irradiation selon l'une quelconque des revendications 1 à 4, **caractérisé en ce que** le module émetteur (11) photonique, de longueur d'ondes prédéterminée, est connecté à une source de courant continu (AL) par un transistor (TR) pour former un circuit de découpage (14).

6. Dispositif d'irradiation selon la revendication 5, **caractérisé en ce que** le circuit de découpage (14) comporte un circuit logique (ET) aux entrées duquel sont connectés un premier oscillateur (12) à basse fréquence (BF) et un deuxième oscillateur (13) à radio fréquence (RF), et que la sortie du circuit logique est branchée à la grille du transistor (TR) pour moduler l'alimentation à double découpage par les deux fréquences (BF et RF) desdits premier et deuxième oscillateurs (12,13).

7. Dispositif d'irradiation selon l'une quelconque des revendications 1 à 6, **caractérisé en ce que** le module émetteur (11) est doté d'une série de diodes, électroluminescentes (LED) ou laser, à rayonnement proche de l'infrarouge.

## Claims

1. A photonic energy irradiation device comprising a nanometric-frequency photonic emitter module (11), **characterized in that** said emitter module (11) is connected to modulating means performing a first chopping at low frequency (LF), and a second chopping at radiofrequency (RF) to obtain a series of low frequency (LF) pulses chopped at radiofrequency (RF).

2. The irradiation device according to claim 1, **characterized in that** the frequency range of the first chopping means at low frequency (LF) is comprised between 5 Hz and 50 Hz, and the frequency range of the second chopping at radiofrequency (RF) is comprised between 500 kHz and 10.000 kHz.

3. The irradiation device according to claim 2, **characterized in that** the frequency range of the second chopping at radiofrequency (RF) is comprised between 1.000 kHz and 1.500 kH.

4. The irradiation device according to one of claims 2 and 3, **characterized in that** the frequency range of the first chopping means at low frequency (LF) is comprised between 10 Hz and 20 Hz

5. The irradiation device according to anyone of claims 1 to 4, **characterized in that** the photonic emitter module (11) of predefined wavelength is connected to a direct current (DC) source by a transistor (TR) to form a chopping circuit (14).

6. The irradiation device according to claim 5, **characterized in that** the chopping circuit (14) comprises a logic AND circuit to the inputs whereof a first oscillator (12) at low frequency (LF) and a second oscillator (13) at radio frequency (RF) are connected to the transistor (TR) gate to modulate the power supply with double chopping by the two frequencies (LF and RF) of said first and second oscillators (12 and 13).

7. The irradiation device according to anyone of claims 1 to 6, **characterized in that** the emitter module (11) is equipped with a series of light-emitting diodes (LED) or laser diodes, with radiation near the infrared.

## Patentansprüche

1. Vorrichtung zur Bestrahlung durch photonische Energiestrahlung, umfassend einem photonischen Ausgabemodul (11) von nanometrischer Wellenlänge, das dadurch charakterisiert ist, dass das Ausgabemodul (11) mit Modulationsmitteln verbunden ist, womit eine erste Partitionierung bei Niedrigfrequenz (NF) und eine zweite Partitionierung bei Radiofrequenz (RF) gegeben ist, um eine Folge von Niedrigfrequenzzeitabständen (BF) zu erhalten, die bei Radiofrequenz (RF) unterteilt sind.

2. Vorrichtung zur Bestrahlung nach Anspruch 1, die dadurch charakterisiert ist, dass der Frequenzbereich der ersten Partitionierung bei Niedrigfrequenz (NF) zwischen 5 und 50 Hz liegt, und der Frequenzbereich der zweiten Partitionierung bei Radiofrequenz (RF) zwischen 500 und 10.000 kHz liegt.

3. Vorrichtung zur Bestrahlung nach Anspruch 2, die dadurch charakterisiert ist, dass der Bereich der zweiten Partitionierung bei Radiofrequenz (RF) zwischen 1.000 und 1.500 kHz liegt.

4. Vorrichtung zur Bestrahlung nach einem der Ansprüche 2 und 3, die dadurch charakterisiert ist, dass der Bereich der ersten Partitionierung bei Niedrigfrequenz (NF) zwischen 10 und 20 Hz liegt.

5. Vorrichtung zur Bestrahlung nach einem beliebigen der Ansprüche 1 bis 4, die dadurch charakterisiert ist, dass das photonische Ausgabemodul (11), von vorbestimmter Wellenlänge, durch einen Transistor (TR) mit einer Gleichstromquelle verbunden ist, um einen Partitionierungskreis (14) zu bilden.

6. Vorrichtung zur Bestrahlung nach Anspruch 5, die dadurch charakterisiert ist, dass die Partitionierungsschaltung (14) mit einer Logikschaltung versehen ist, an deren Eingängen ein erster Niedrigfrequenz-Oszillator (12) und ein zweiter Radiofrequenz-Oszillator (13) angeschlossen sind und dass der Ausgang der Logikschaltung an das Gitter des Transistors (TR) angeschlossen ist, um die Speisung bei doppelter Partitionierung durch die beiden Frequenzen (NF und RF) der besagten ersten und zweiten Oszillatoren (12, 13) zu modulieren.

7. Vorrichtung zur Bestrahlung nach einem beliebigen der Ansprüche 1 bis 6, die dadurch charakterisiert ist, dass das Ausgabemodul (11) mit einer Serie von LED- oder Laser-Dioden ausgerüstet ist, deren Strahlung einer Infrarot-Strahlung nahekommt.
